(19)

Europäisches Patentamt
European Patent Office
Office européen des brevets

(11) **EP 4 083 133 A1**

(12) **EUROPEAN PATENT APPLICATION**
published in accordance with Art. 153(4) EPC

(43) Date of publication:
02.11.2022 Bulletin 2022/44

(21) Application number: 20907506.8

(22) Date of filing: 24.12.2020

(51) International Patent Classification (IPC):
$C08L\ 11/02^{(2006.01)}$    $A41D\ 19/00^{(2006.01)}$
$A41D\ 19/04^{(2006.01)}$    $A61B\ 42/10^{(2016.01)}$
$B29C\ 41/14^{(2006.01)}$    $B29C\ 41/36^{(2006.01)}$
$C08F\ 236/18^{(2006.01)}$    $C08K\ 3/06^{(2006.01)}$
$C08K\ 3/22^{(2006.01)}$    $C08K\ 5/39^{(2006.01)}$
$C08K\ 5/47^{(2006.01)}$

(52) Cooperative Patent Classification (CPC):
B29C 41/14; A61B 42/10; B29C 41/22; C08K 3/06;
C08K 3/22; C08K 5/39; C08K 5/47; B29K 2011/00;
B29K 2105/0064; B29L 2031/4864

(86) International application number:
PCT/JP2020/048437

(87) International publication number:
WO 2021/132460 (01.07.2021 Gazette 2021/26)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 24.12.2019 JP 2019233053

(71) Applicant: Showa Denko K.K.
Tokyo 105-8518 (JP)

(72) Inventors:
• KANEKO Shu
Tokyo 105-8518 (JP)
• OGAWA Masahiro
Tokyo 105-8518 (JP)
• SHIBUYA Akira
Tokyo 105-8518 (JP)

(74) Representative: Strehl Schübel-Hopf & Partner
Maximilianstrasse 54
80538 München (DE)

(54) **CHLOROPRENE COPOLYMER LATEX COMPOSITION AND MOLDED ARTICLE OF SAME**

(57) The present invention relates to a chloroprene copolymer latex composition, a molded article of chloroprene copolymer rubber, or a dip-molded product, and the chloroprene copolymer latex composition includes a chloroprene copolymer (A) and a vulcanization accelerator (B). The chloroprene copolymer (A) includes monomer units derived from chloroprene and from 2-methyl-1,3-butadiene. The chloroprene copolymer latex composition includes a carbamate-based vulcanization accelerator and a thiazole-based vulcanization accelerator as the vulcanization accelerator (B). The chloroprene copolymer latex composition enables production of a molded article having high flexibility and high tensile strength without use of a vulcanization accelerator that is feared to have skin sensitization.

EP 4 083 133 A1

**Description**

Technical Field

The present invention relates to a latex composition including, as a main component, a copolymer of 2-chloro-1,3-butadiene (chloroprene) and 2-methyl-1,3-butadiene, and a molded article, particularly a dip-molded product, using a composition including the latex.

Background Art

Isoprene rubber (IR) and chloroprene rubber (CR) are synthetic rubber having flexibility equivalent to that of natural rubber. Thus, isoprene rubber or chloroprene rubber has been recently used, instead of natural rubber, in a material for products obtained by dip-molding a composition (dip-molded products), especially surgical gloves, as a countermeasure against allergy. Isoprene rubber has high flexibility and is likely to follow hand movements. Gloves produced from isoprene rubber tend to provide a favorable feeling of use (tactile sensation) for medical practitioners, but isoprene rubber does not fully meet the needs of the market because of its high cost. Meanwhile, chloroprene rubber can be produced more inexpensively than isoprene rubber. However, when flexibility is imparted to chloroprene rubber, which is inferior in flexibility to isoprene rubber, problems arise such as decrease in the tensile strength.

With respect to chloroprene rubber, a technique to improve the flexibility is disclosed in Patent Literature 1, for example, but the technique decreases the tensile strength. A technique to impart high strength by means of a production method or blending for vulcanization is disclosed in Patent Literature 2, for example. However, the technique decreases the flexibility, and the flexibility and high strength are not sufficiently achieved in combination.

Citation List

Patent Literature

Patent Literature 1: Japanese Patent Laid-Open No. 2019-143002
Patent Literature 2: Japanese Patent Laid-Open No. 2019-044116

Summary of Invention

Technical Problem

It is an object of the present invention to solve the problems of the conventional arts and to inexpensively provide a chloroprene copolymer latex that can provide a molded article having excellent flexibility. It is another object of the present invention to provide a chloroprene copolymer latex composition without use of diphenylguanidine or N,N'-diphenylthiourea, which are being recognized as sensitizing substances in Europe.

Solution to Problem

The present inventors have intensively studied to solve the above problems and, as a result, the inventors have found that the above problems can be solved by employing a latex of (A) a chloroprene copolymer including monomer units derived from chloroprene and monomer units derived from 2-methyl-1,3-butadiene and using a thiazole-based vulcanization accelerator and a carbamate-based vulcanization accelerator as vulcanization accelerators, and thus have completed the present invention.

That is, the present invention relates to a chloroprene copolymer latex composition, a molded article provided by curing the composition, and a dip-molded product.

[1] A chloroprene copolymer latex composition comprising a chloroprene copolymer (A) and a vulcanization accelerator (B), wherein

the chloroprene copolymer (A) is a chloroprene copolymer comprising monomer units derived from chloroprene and from 2-methyl-1,3-butadiene, and
the vulcanization accelerator (B) comprises a carbamate-based vulcanization accelerator and a thiazole-based vulcanization accelerator.

[2] The chloroprene copolymer latex composition according to [1], wherein the thiazole-based vulcanization accelerator is zinc 2-mercaptobenzothiazole.

[3] The chloroprene copolymer latex composition according to [1] or [2], wherein the carbamate-based vulcanization accelerator is at least one selected from zinc diethyldithiocarbamate, zinc dibutyldithiocarbamate, and sodium dibutyldithiocarbamate.

[4] The chloroprene copolymer latex composition according to any of [1] to [3], wherein the chloroprene copolymer latex composition further comprises a metal oxide (C), sulfur (D), and an antioxidant (E).

[5] A chloroprene copolymer latex composition according to [4], comprising:

100 parts by mass of the chloroprene copolymer (A) and an optional synthetic rubber (F) in total;
0.1 to 10.0 parts by mass of the vulcanization accelerator (B);
0.1 to 20.0 parts by mass of the metal oxide (C);
0.1 to 10.0 parts by mass of the sulfur (D); and
0.1 to 10.0 parts by mass of the antioxidant (E).

[6] The chloroprene copolymer latex composition according to [5], comprising a synthetic rubber (F), wherein a proportion of the synthetic rubber (F) is 1 to 33% by mass with respect to 100% by mass of a total of the chloroprene copolymer (A) and the synthetic rubber (F).

[7] A molded article of a chloroprene copolymer rubber, provided by curing the chloroprene copolymer latex composition according to any of [1] to [6].

[8] A dip-molded product provided by molding the chloroprene copolymer latex composition according to any of [1] to [6] by a dipping method followed by curing.

[9] The dip-molded product according to [8], wherein the dip-molded product is gloves.

[10] The dip-molded product according to [9], wherein the dip-molded product is medical disposable gloves.

[11] The dip-molded product according to any of [8] to [10], wherein no powder is present on a surface of the dip-molded product, the powder being for alleviating friction between the dip-molded product and an object to be in contact with the dip-molded product.

[12] A multilayer dip-molded product having a multilayer structure comprising:

a layer provided by molding the chloroprene copolymer latex composition according to any of [1] to [6] by a dipping method followed by curing; and
another layer thereon.

Advantageous Effect of Invention

Vulcanizing the chloroprene copolymer latex composition of the present invention can provide a molded article having high flexibility and high tensile strength (molded article of a chloroprene copolymer rubber). The chloroprene copolymer latex composition of the present invention does not contain a vulcanization accelerator that is feared to have skin sensitization. Thus, molded articles produced from the chloroprene copolymer latex composition of the present invention can be suitably used for dip-molded products, particularly medical disposable gloves.

Description of Embodiment

Hereinafter, embodiments of the present invention will be described in detail, but the present invention is not limited to the configurations of the following embodiments. In the statements herein and also in claims, "to" indicating a numerical range means numerical values between the lower limit and the upper limit of the range, both inclusive.

<<Chloroprene copolymer latex composition>>

The chloroprene copolymer latex composition of the present invention includes a chloroprene copolymer (A) and a vulcanization accelerator (B). Hereinafter, a case where a latex of the chloroprene copolymer (A) and the vulcanization accelerator (B) are mixed to produce a chloroprene copolymer latex composition will be described as an example.

<Latex of chloroprene copolymer (A)>

In a latex of the chloroprene copolymer (A), particulates of the chloroprene copolymer (A) are dispersed in a dispersion medium such as water.

[Chloroprene copolymer (A)]

The chloroprene copolymer (A) includes at least structures (monomer units) derived from 2-chloro-1,3-butadiene (chloroprene) (A-1) and from 2-methyl-1,3-butadiene (A-2). The monomer constituting the chloroprene copolymer (A) may be only 2-chloro-1,3-butadiene (A-1) and 2-methyl-1,3-butadiene (A-2).

The proportion of the monomer units derived from 2-chloro-1,3-butadiene (A-1) is preferably 70 to 90 mol%, more preferably 73 to 90 mol%, still more preferably 75 to 89 mol%, particularly preferably 84 to 89 mol%, with respect to 100 mol% of the total monomer units constituting the chloroprene copolymer (A).

A proportion of the monomer units derived from 2-chloro-1,3-butadiene (A-1) in the chloroprene copolymer (A) of 73 mol% or more is preferred because the polymerization reaction tends to progress fast. A proportion of the monomer units derived from 2-chloro-1,3-butadiene (A-1) in the chloroprene copolymer (A) of 90 mol% or less is preferred because a molded article to be provided by vulcanization treatment of the chloroprene copolymer latex composition has high flexibility.

The proportion of 2-methyl-1,3-butadiene (A-2) is preferably 10 to 30 mol%, more preferably 10 to 27 mol%, further preferably 11 to 25 mol%, particularly preferably 11 to 16 mol%, with respect to 100 mol% of the total monomer units constituting the chloroprene copolymer (A). The proportion of 2-methyl-1,3-butadiene (A-2) is determined by $^1$H-NMR analysis described in examples.

A proportion of the monomer units derived from 2-methyl-1,3-butadiene (A-2) in the chloroprene copolymer (A) of 10 to 30 mol% is preferred because a molded article provided on vulcanization at 110°C has a favorable tensile strength.

The chloroprene copolymer (A) can include monomer units derived from the monomer (A-3) as long as the object of the present invention is not impaired, in addition to the structures (monomer units) derived from 2-chloro-1,3-butadiene (A-1) and the structures (monomer units) derived from 2-methyl-1,3-butadiene (A-2). Here, the monomer (A-3) is a monomer other than 2-chloro-1,3-butadiene (A-1) or 2-methyl-1,3-butadiene (A-2), and is copolymerizable with at least one of 2-chloro-1,3-butadiene (A-1) and 2-methyl-1,3-butadiene (A-2). The monomer (A-3) may be a monomer copolymerizable with both 2-chloro-1,3-butadiene (A-1) and 2-methyl-1,3-butadiene (A-2). Examples of the monomer (A-3) include butadiene, 2,3-dichloro-1,3-butadiene, styrene, acrylonitrile, acrylic acid and esters thereof, and methacrylic acid and esters thereof. The chloroprene copolymer (A) may include, as required, structures derived from two or more monomers (A-3).

In the case where the chloroprene copolymer (A) contains units of the monomers (A-3), the proportion (upper limit) of the monomers (A-3) is preferably 10 parts by mole or less, more preferably 8 parts by mole or less, still more preferably 5 parts by mole or less, per 100 parts by mole of the total of the monomer units derived from 2-chloro-1,3-butadiene (A-1) and the monomer units derived from 2-methyl-1,3-butadiene (A-2) in the chloroprene copolymer (A). In the case where the chloroprene copolymer (A) contains units of the monomers (A-3), the proportion (lower limit) of the monomers (A-3) is preferably 0.01 parts by mole or more, more preferably 0.5 parts by mole or more, still more preferably 1.0 parts by mole or more, per 100 parts by mole of the total of the monomer units derived from 2-chloro-1,3-butadiene (A-1) and the monomer units derived from 2-methyl-1,3-butadiene (A-2) in the chloroprene copolymer (A). When the proportion of the monomers (A-3) is 10 parts by mole or less per 100 parts by mole of the total of the monomer units derived from 2-chloro-1,3-butadiene (A-1) and the monomer units derived from 2-methyl-1,3-butadiene (A-2) in the chloroprene copolymer (A), the tensile strength and elongation of a molded article are favorable, and the stability over time of the flexibility of the molded article is favorable.

The tetrahydrofuran (THF) insoluble content at 25°C of the chloroprene copolymer (A) is preferably 30% by mass or less, more preferably 20% by mass or less, further preferably 10% by mass or less. A tetrahydrofuran insoluble content of the chloroprene copolymer (A) of 30% by mass or less is preferred because of favorable flexibility and tensile strength of a molded article to be provided by vulcanization treatment.

The tetrahydrofuran insoluble content of the chloroprene copolymer (A) is preferably 0.01% by mass or more, more preferably 0.1% by mass or more, further preferably 1.5% by mass or more. A tetrahydrofuran insoluble content of the chloroprene copolymer (A) of 0.01% by mass or more is preferred because crosslinking in the chloroprene copolymer (A) has progressed.

The tetrahydrofuran insoluble content is a gelled product of polymer chains via three-dimensional crosslinking in chloroprene copolymer particles. The tetrahydrofuran insoluble content can be measured by a method employed in examples described below.

A tetrahydrofuran insoluble content of the chloroprene copolymer (A) can be controlled by adjusting the polymerization conversion and the amount of chain transfer agent. The polymerization conversion can be controlled via the polymerization time and polymerization temperature of the chloroprene copolymer (A). A longer polymerization time tends to lead to a higher polymerization conversion, and a higher polymerization temperature tends to lead to a higher polymerization conversion.

For example, an increase in the polymerization conversion tends to increase the tetrahydrofuran insoluble content of the chloroprene copolymer (A). An increase in the amount of the chain transfer agent present on emulsion polymerization of the chloroprene copolymer (A) tends to reduce the tetrahydrofuran insoluble content of the chloroprene copolymer (A).

The weight average molecular weight of the tetrahydrofuran soluble component at 25°C of the chloroprene copolymer (A) is preferably 400,000 or more, more preferably 500,000 or more, still more preferably 550,000, as measured by the method or conditions employed in examples described later. When the weight average molecular weight of the tetrahydrofuran soluble component at 25°C of the chloroprene copolymer (A) is 400,000 or more, a molded article having favorable mechanical properties can be provided. The weight average molecular weight of the tetrahydrofuran soluble component at 25°C of the chloroprene copolymer (A) is preferably 3,000,000 or less, more preferably 2,000,000 or less, still more preferably 900,000 or less. When the weight average molecular weight of the tetrahydrofuran soluble component at 25°C of the chloroprene copolymer (A) is 3,000,000 or less, the tetrahydrofuran insoluble content can fall within a desired range, and a molded article having favorable flexibility and tensile strength can be provided.

[Method for producing latex of chloroprene copolymer (A)]

As a method for producing the latex of the chloroprene copolymer (A), a method of radically polymerizing 2-chloro-1,3-butadiene (A-1) and 2-methyl-1,3-butadiene (A-2) in an aqueous emulsion is simple and industrially advantageous.

Emulsion polymerizing 2-chloro-1,3-butadiene (A-1) and 2-methyl-1,3-butadiene (A-2) or 2-chloro-1,3-butadiene (A-1), 2-methyl-1,3-butadiene (A-2), and the monomers (A-3) using an emulsifier can provide a latex including particles of the chloroprene copolymer (A) dispersed in a dispersion medium such as water. The polymerization temperature on the emulsion polymerization is preferably 20 to 35°C, and the polymerization time is preferably 5 to 8 hours. The polymerization temperature and polymerization time on the emulsion polymerization are preferably within the above ranges because a desired polymerization conversion is achieved.

The content of 2-methyl-1,3-butadiene in the chloroprene copolymer (A) can be adjusted by means of, for example, the proportions of 2-chloro-1,3-butadiene (A-1) and 2-methyl-1,3-butadiene (A-2) fed for polymerization and the polymerization conversion thereof.

A higher proportion of 2-methyl-1,3-butadiene (A-2) to the total monomers on polymerization feeding can finally result in a large content of the monomer units derived from 2-methyl-1,3-butadiene (A-2) in the chloroprene copolymer (A). However, 2-methyl-1,3-butadiene (A-2) has lower reactivity at the beginning of the emulsion polymerization than 2-chloro-1,3-butadiene (A-1). Thus, a larger proportion of 2-methyl-1,3-butadiene (A-2) fed tends to retard the progress of the polymerization and lengthen the reaction time.

As the polymerization of the chloroprene copolymer (A) proceeds, 2-methyl-1,3-butadiene (A-2) is more likely to be incorporated in the polymer. Then, an increase in the polymerization conversion on polymerization for the chloroprene copolymer (A) can lead to an increase in the content of the monomer units derived from 2-methyl-1,3-butadiene (A-2) with respect to the final chloroprene copolymer (A). With a low polymerization conversion, remaining monomers increase, which requires complicated removal of the remaining monomer, and moreover, mechanical properties of the molded article are degraded.

In view of the above, the content of 2-methyl-1,3-butadiene (A-2) in the total monomer components used is preferably 2 to 40 mol%, more preferably 10 to 30 mol%, still more preferably, 15 to 25 mol%, in view of effectively providing the chloroprene copolymer (A) in the present invention. The polymerization conversion of the total monomers is preferably 61 to 90% by mass, more preferably 75 to 87% by mass, still more preferably 80 to 86% by mass. When the polymerization conversion of the total monomers is 90% by mass or less, the quality of the chloroprene copolymer (A) provided by the polymerization is favorable, and the physical properties of a molded article provided from the latex of the chloroprene copolymer (A) are also favorable.

The emulsifier for the emulsion polymerization is preferably an anionic surfactant. Examples of the anionic surfactant include rosin acid soap, sodium salts of naphthalenesulfonic acid condensates, sodium salts of dodecylbenzenesulfonic acid, and sodium salts of dodecylsulfuric acid. Usual rosin acid soap can be used in view of simple operation for solidification. Particularly in view of coloring stability, a sodium salt and/or potassium salt of disproportionated rosin acid can be used. In view of the polymerization rate, a potassium salt of disproportionated rosin acid is more preferred.

The amount of the emulsifier used is 0.5 to 20.0 parts by mass, more preferably 1.0 to 10.0 parts by mass, still more preferably 1.5 to 5.0 parts by mass, per 100 parts by mass of the total of all the monomers: 2-chloro-1,3-butadiene (A-1), 2-methyl-1,3-butadiene (A-2), and the monomer (A-3). When the amount of the emulsifier used is 0.5 parts by mass or more, poor emulsification is unlikely to occur, and exotherm due to the polymerization can be controlled. When the amount of the emulsifier used is 0.5 parts by mass or more, problems such as generation of aggregates and poor appearance of products do not arise. On the other hand, when the amount of the emulsifier used is 20.0 parts by mass or less, the emulsifier such as rosin acid does not remain in the chloroprene copolymer (A), and adhesion is unlikely to occur in the chloroprene copolymer (A). Thus, when the amount of the emulsifier used is 20.0 parts by mass or less, problems of processability and handleability due to, for example, adhesion of the chloroprene copolymer latex composition to the mold (former) on molding or adhesion of a molded article on use does not occur, and the color tone of the molded article does not deteriorate.

As a polymerization initiator, a usual radical polymerization initiator can be used. For example, an organic or inorganic peroxide such as benzoyl peroxide, potassium peroxide, ammonium persulfate, cumene hydroperoxide, and t-butyl hydroperoxide, or an azo compound such as azobisisobutyronitrile is used in the case of emulsion polymerization. One of the polymerization initiators may be used singly, or two or more thereof may be used in combination.

In polymerization of the chloroprene copolymer (A) of the present embodiment, a chain transfer agent is preferably used for adjusting the amount of the tetrahydrofuran insoluble content. The amount of the chain transfer agent used is preferably 0.01 to 15.0 parts by mass, more preferably 0.05 to 10.0 parts by mass, still more preferably 0.1 to 1.0 parts by mass, per 100 parts by mass of the total of all the monomers: 2-chloro-1,3-butadiene (A-1), 2-methyl-1,3-butadiene (A-2), and the monomer (A-3).

The chain transfer agent is not particularly limited, and a known chain transfer agent can be used, including an alkylmercaptan such as n-dodecylmercaptan, n-decylmercaptan, octylmercaptan, or tert-dodecylmercaptan, a dialkyl xanthogen disulfide such as diisopropyl xanthogen disulfide or diethyl xanthogen disulfide, or iodoform. More preferred is an alkylmercaptan, and still more preferred is n-dodecylmercaptan.

Setting the polymerization conversion to 60 to 90% by mass and the amount of the chain transfer agent used to 0.01 to 15.0 parts by mass can adjust the tetrahydrofuran insoluble content of the chloroprene copolymer (A) within a desired range (30% by mass or less).

In polymerization of the chloroprene copolymer (A), a cocatalyst may be used with the polymerization initiator, if desired. The cocatalyst that can be used with the polymerization initiator is not particularly limited, and a common cocatalyst can be used. Examples of the cocatalyst include anthraquinonesulfonates, potassium sulfite, sodium disulfite, sodium sulfite, tetraethylenepentamine, and N,N-dimethyl-p-toluidine. One of the cocatalysts may be used singly, or two or more thereof may be used in combination.

Generally in emulsion polymerization, a polymerization terminator is added when a predetermined polymerization conversion is reached to thereby stop the polymerization reaction, in order to provide a polymer having a desired molecular weight and a desired molecular weight distribution. A polymerization terminator may be used also in the embodiment of the present invention. The type of polymerization terminator is not particularly limited, and a polymerization terminator usually used can be used, including phenothiazine, para-t-butylcatechol, hydroquinone, hydroquinone monomethylether, and diethylhydroxylamine. One of the polymerization terminators may be used singly, or two or more thereof may be used in combination.

In addition, a stabilizer such as an acid acceptor and/or an antioxidant may be blended to the latex of the chloroprene copolymer (A) as long as the object of the present invention is not impaired.

[Chloroprene copolymer latex composition]

The chloroprene copolymer latex composition in one embodiment of the present invention includes a chloroprene copolymer (A), a vulcanization accelerator (B), and, as optional components, a metal oxide (C), sulfur (D), an antioxidant (E), and synthetic rubber (F). The solid content of the latex of the chloroprene copolymer (A) and the chloroprene copolymer latex composition here refer to a component provided when allowing the latex of the chloroprene copolymer (A) or the chloroprene copolymer latex composition to stand in an oven at 141°C for 30 minutes for drying. The component is provided by removing the dispersion medium such as water from the latex. The chloroprene copolymer latex composition may contain a dispersion medium such as water derived from the latex of the chloroprene copolymer (A).

In the case of not including synthetic rubber (F) described below, the chloroprene copolymer latex composition preferably further includes 1.0 to 10.0 parts by mass of the vulcanization accelerator (B), per 100 parts by mass of the solid content in the latex of the chloroprene copolymer (A). In order to conduct vulcanization treatment efficiently, the composition preferably includes 0.1 to 20.0 parts by mass of the metal oxide (C), 0.1 to 10.0 parts by mass of the sulfur (D), and 0.1 to 10.0 parts by mass of the antioxidant (E), per 100 parts by mass of the solid content of the latex of the chloroprene copolymer (A). Vulcanizing the chloroprene copolymer latex composition prepared in this formulation provides a safe rubber molded article (e.g., a film) efficiently. Among the materials used for blending, a water-insoluble component and a component that destabilizes the colloid state of the latex of the chloroprene copolymer (A) are each made into an aqueous dispersion in advance, and then the aqueous dispersion is added to the latex of the chloroprene copolymer (A).

The vulcanization accelerator (B) includes a thiazole-based vulcanization accelerator and a carbamate-based vulcanization accelerator. Examples of the thiazole-based vulcanization accelerator include 2-mercaptobenzothiazole, di-2-benzothiazolyl disulfide, and zinc 2-mercaptobenzothiazole. Examples of the carbamate-based vulcanization accelerator include zinc dimethyldithiocarbamate, zinc diethyldithiocarbamate, zinc dibutyldithiocarbamate, zinc N-ethyl-N-phenyldithiocarbamate, sodium dibutyldithiocarbamate, zinc N-pentamethylenedithiocarbamate, zinc dibenzyldithiocarbamate, copper dimethyldithiocarbamate, and tellurium diethyldithiocarbamate. In order to conduct vulcanization treatment efficiently, sodium dibutyldithiocarbamate, zinc diethyldithiocarbamate, or zinc dibutyldithiocarbamate is preferably used. Three or more of these vulcanization accelerator may be used in combination.

For the vulcanization accelerator (B), a further vulcanization accelerator may be used in combination, as required. The type of the vulcanization accelerator to be used in combination is not particularly limited, and it is possible to use a vulcanization accelerator commonly used for vulcanization treatment of an isoprene-based polymer latex or a chloroprene-based polymer latex. Examples thereof include thiuram-based vulcanization accelerators, thiourea-based vulcanization accelerators, and guanidine-based vulcanization accelerators. Examples of the thiuram-based vulcanization accelerator include tetraethylthiuram disulfide and tetrabutylthiuram disulfide. Examples of the thiourea-based vulcanization accelerator include ethylene thiourea, diethyl thiourea, trimethyl thiourea, and N,N'-diphenyl thiourea (DPTU). Examples of the guanidine-based vulcanization accelerator include diphenyl guanidine (DPG) and diorthotoluyl guanidine. These may be used singly, or in combinations of two or more thereof.

The amount of the vulcanization accelerator (B) contained in the chloroprene copolymer latex composition according to the present embodiment is preferably 1.0 to 10.0 parts by mass, more preferably 1.2 to 5.0 parts by mass, still more preferably 1.5 to 3.0 parts by mass, per 100 parts by mass of the solid content of the latex of the chloroprene copolymer (A) provided by the polymerization method described above. When the amount of the vulcanization accelerator (B) is within this range, a moderate vulcanization rate can be achieved, lack of crosslinked structures due to insufficient vulcanization is unlikely to occur, and additionally, scorching is unlikely to occur. Also, when setting the amount of the vulcanization accelerator (B) within the above range, a molded article provided from the chloroprene copolymer latex composition according to the present embodiment has a moderate vulcanization density, and the mechanical properties of the molded article are thus allowed to fall within appropriate ranges.

The type of the metal oxide (C) is not particularly limited. Examples thereof that can be used include zinc oxide, lead oxide, and trilead tetraoxide, and zinc oxide is particularly preferred. One of the metal oxides (C) may be used singly, or two or more thereof may be used in combination.

The amount of the metal oxide (C) contained in the chloroprene copolymer latex composition according to the present embodiment is preferably 0.1 to 20.0 parts by mass, more preferably 0.25 to 15.0 parts by mass, still more preferably 0.4 to 10.0 parts by mass, per 100 parts by mass of the solid content in the latex of the chloroprene copolymer (A). An amount of the metal oxide (C) of 0.1 parts by mass or more is preferred because a moderate vulcanization rate can be achieved. When the amount of the metal oxide (C) is 20.0 parts by mass or less, a favorable crosslinked structure is provided by the vulcanization treatment, and scorching is unlikely to occur. The amount is also preferred because of the following: the colloid state of the chloroprene copolymer latex composition is stabilized, and thus problems such as precipitation are unlikely to arise.

The type of the sulfur (D) is not particularly limited. Powdered sulfur, precipitated sulfur, colloidal sulfur, surface-treated sulfur, and insoluble sulfur, as well as sulfur-containing compounds such as polysulfides and polymeric polysulfides (except for the above vulcanization accelerators) can be used. One of the sulfurs (D) may be used singly, or two or more thereof may be used in combination. The amount of the sulfur (D) contained in the chloroprene copolymer latex composition according to the present embodiment is preferably 0.1 to 10.0 parts by mass, more preferably 0.2 to 7.0 parts by mass, still more preferably 0.8 to 5.0 parts by mass, per 100 parts by mass the solid content in the latex of the chloroprene copolymer (A). An amount of the sulfur (D) within this range is preferred because of the following: a moderate vulcanization rate can be achieved, lack of crosslinked structures due to insufficient vulcanization treatment is unlikely to occur, and additionally, scorching is unlikely to occur. This is also preferred because the colloid state of the chloroprene copolymer latex composition is stabilized, and thus, problems such as precipitation are unlikely to occur.

The type of the antioxidant (E) is not particularly limited. When a molded article having high heat resistance is desirable, an antioxidant that prevents thermal aging and an antioxidant that prevents ozone aging are preferably used in combination.

Examples of the antioxidant that prevents thermal aging include diphenylamine-based antioxidants such as octylated diphenylamine, p-(p-toluene-sulfonylamide) diphenylamine, and 4,4'-bis($\alpha,\alpha$-dimethylbenzyl) diphenylamine. Blending such an antioxidant tends to allow the molded article to have heat resistance and also have contamination resistance (e.g., inhibition of discoloration).

Examples of the antioxidant that prevents ozone aging include N,N'-diphenyl-p-phenylenediamene (DPPD) and N-isopropyl-N'-phenyl-p-phenylenediamene (IPPD).

When the molded article of a chloroprene copolymer rubber according to the present embodiment is used as medical disposable gloves, appearances (in particular, color tone) and hygiene are considered important. Thus, as the antioxidant (E), a hindered phenolic antioxidant is preferably used. Examples of the hindered phenolic antioxidant include 2,2'-methylenebis-(4-ethyl-6-t-butylphenol) and 4,4'-methylenebis-(2,6-di-t-butylphenol).

The amount of the antioxidant (E) contained in the chloroprene copolymer latex composition according to the present embodiment is preferably 0.1 to 10.0 parts by mass, more preferably 0.5 to 5.5 parts by mass, still more preferably 1.0 to 4.8 parts by mass, per 100 parts by mass of the solid content in the latex of the chloroprene copolymer (A). When the amount of the antioxidant (E) is within this range, a sufficient antioxidant effect is provided while the vulcanization treatment is not inhibited, and the color tone is unlikely to deteriorate.

The chloroprene copolymer latex composition can include synthetic rubber (F) miscible with the latex of the chloroprene copolymer (A). The chloroprene copolymer latex composition preferably contains the synthetic rubber (F) because other rubber properties that are not possessed by the chloroprene copolymer (A) can be imparted to a molded article. Miscible synthetic rubber (F) is not particularly limited and may be selected from isoprene rubber, chloroprene rubber (except for the chloroprene copolymer (A)), acrylonitrile-butadiene rubber, and butadiene rubber, for example. In respect of compatibility with the chloroprene copolymer (A), isoprene rubber or chloroprene rubber (except for the chloroprene copolymer (A)) is more preferred. Two or more synthetic rubbers (F) may be used, as required, in the chloroprene copolymer latex composition.

The synthetic rubber (F) in the chloroprene copolymer latex composition may be blended in an amount that is not contrary to the objects of the present invention. In the case where the chloroprene copolymer latex composition includes miscible synthetic rubber (F), the proportion (upper limit) of the synthetic rubber (F) is preferably 25% by mass or less, more preferably 10% by mass or less, with respect to 100% by mass of the total of the solid content of the latex of the chloroprene copolymer (A) and the synthetic rubber (F). The proportion (lower limit) of the synthetic rubber (F) is preferably 1% by mass or more, more preferably 3% by mass or more, still more preferably 5% by mass or more. A proportion of the synthetic rubber (F) of 25% by mass or less is preferred because the maturing time and/or vulcanization time of the chloroprene copolymer latex composition are/is short. A proportion of the synthetic rubber (F) of 10% by mass or more is preferred because the properties of the other synthetic rubber (F) are developed.

The amount of the synthetic rubber (F) blended to the chloroprene copolymer latex composition is preferably 33 parts by mass or less, more preferably 11 parts by mass or less, with respect to 100 parts by mass of the chloroprene copolymer (A). The amount of the synthetic rubber (F) blended is preferably 1 part by mass or more, more preferably 3.1 parts by mass or more, still more preferably 5.3 parts by mass or more, with respect to 100 parts by mass of the chloroprene copolymer (A).

In the case where the chloroprene copolymer latex composition includes the chloroprene copolymer (A) and the synthetic rubber (F), the chloroprene copolymer latex composition may include 0.1 to 10.0 parts by mass of the vulcanization accelerator (B), 0.1 to 20.0 parts by mass of the metal oxide (C), 0.1 to 10.0 parts by mass of the sulfur (D), and 0.1 to 10.0 parts by mass of the antioxidant (E), per 100 parts by mass of the total of the solid content of the latex of the chloroprene copolymer (A) and the synthetic rubber (F).

The synthetic rubber (F) may be a latex including particulates of the synthetic rubber (F) dispersed therein. In the case where a latex of the synthetic rubber (F) is employed, the chloroprene copolymer latex composition includes 0.1 to 10.0 parts by mass of the vulcanization accelerator (B), 0.1 to 20.0 parts by mass of the metal oxide (C), 0.1 to 10.0 parts by mass of the sulfur (D), and 0.1 to 10.0 parts by mass of the antioxidant (E), per 100 parts by mass of total of the solid content of the latex of the chloroprene copolymer (A) and the solid content of the latex of the synthetic rubber (F).

To the chloroprene copolymer latex composition according to the present embodiment, other additives may be blended, as desired, in addition to the chloroprene copolymer (A), the vulcanization accelerator (B), the metal oxide (C), the sulfur (D), the antioxidant (E), and the synthetic rubber (F) as long as the other additives are not contrary to the objects of the present invention. Examples of the additives that can be blended include a pH adjuster, a filler, a pigment, a colorant, an antifoaming agent, and a thickener.

[Molded article of chloroprene copolymer rubber]

The chloroprene copolymer latex composition according to the present invention can be molded or cured to thereby provide a molded article of a chloroprene copolymer rubber. For example, the chloroprene copolymer latex composition can be molded by a dip processing method to thereby provide a dip-molded product.

The chloroprene copolymer latex composition may be matured under predetermined conditions before the dip processing. The temperature conditions for the maturing is 15 to 40°C, and the maturing time is 15 to 72 hours. For example, conditions of maturing at 23°C for 20 hours may be employed. The starting point of the maturing is the time point when the latex of the chloroprene copolymer latex (A) is mixed with all of the vulcanization accelerator (B), the metal oxide (C), the sulfur (D), and the antioxidant (E).

After the chloroprene copolymer latex composition is matured, the steps of a dip and solidification treatment, drying, and vulcanization treatment (curing) are conducted in this order to thereby provide a molded article in a film form.

The dip and solidification treatment can be conducted by submerging a plate or mold coated with a coagulant in the chloroprene copolymer latex composition for a predetermined time to thereby deposit the solid content in the chloroprene copolymer latex composition, including the chloroprene copolymer (A), on the surface of the plate or mold. This is probably because of the following: particulates covered with a film of an emulsifier having surface activity, for example are formed in the chloroprene copolymer latex composition; the film on the particulates is collapsed by the action of a coagulant adhering to the surface of a plate or mold; and the chloroprene copolymer (A), for example, in the particulates adheres to the surface of the plate or mold. As the coagulant, a metal salt can be used. For example, a metal nitrate can be used.

In order to avoid the problem of the appearance of the molded article, such as generation of a blister or pinhole, a drying step at a relatively low temperature of 70°C or more and 100°C or less (roughly drying step) may be conducted before the vulcanization step.

The vulcanization temperature in the vulcanization step can be 110 to 130°C. For example, the solid content of the chloroprene copolymer latex composition deposited by a dip and solidification treatment can be vulcanized at 110°C in air. The vulcanization time at this vulcanization temperature range can be 20 minutes or more and 90 minutes or less, for example. Sufficient vulcanization treatment is preferably conducted to the extent that the tensile strength and tensile elongation ratio of the molded article do not deteriorate.

Vulcanizing the composition deposited on the surface of the plate or mold under the above conditions can provide a molded article of a chloroprene copolymer rubber. The molded article of a chloroprene copolymer rubber preferably has a 500% elastic modulus of 0.5 to 1.6 MPa, a tensile strength of 19 to 30 MPa, and a tensile elongation ratio of 800 to 1500%. The molded article of a chloroprene copolymer rubber according to the present embodiment has excellent flexibility and also has a tensile strength within a desired range.

[Medical disposable gloves]

The molded article of a chloroprene copolymer rubber can be suitably used particularly as medical disposable gloves.

The molded article of a chloroprene copolymer rubber has preferably a 100% elastic modulus of 0.60 MPa or less because flexibility is achieved in medical disposable gloves. Regarding the lower limit, the 100% elastic modulus of the molded article of a chloroprene copolymer rubber may be 0.40 MPa or more, for example.

When the molded article of a chloroprene copolymer rubber has a 500% elastic modulus of 0.5 to 1.6 MPa, the medical disposable gloves has a soft feeling of use. Thus, users are unlikely to be tired even if using the gloves for a long period. The molded article of a chloroprene copolymer rubber preferably has a 500% elastic modulus of 1.6 MPa or less because the force to return is appropriate when fingers are bent in the medical disposable gloves.

When the molded article of a chloroprene copolymer rubber preferably has a tensile strength of 19 MPa or more, a sufficient strength for medical disposable gloves is achieved, and breaks of the gloves are unlikely to occur. Regarding the upper limit of the tensile strength of the molded article of a chloroprene copolymer rubber may be 30 MPa or less, for example.

The molded article of a chloroprene copolymer rubber preferably has a tensile elongation ratio of 800% or more because breaks of the medical disposable gloves are unlikely to occur. Regarding the upper limit, the tensile elongation ratio of the molded article of a chloroprene copolymer rubber may be 1500% or less, for example.

In the case where the molded article of a chloroprene copolymer rubber is used as a dip-molded product, powder such as calcium carbonate or corn starch may be applied to the surface of the dip-molded product in order to alleviate friction between the dip-molded product and an object to be in contact with the dip-molded product. Here, the object may be an article with which the dip-molded product comes in contact or may be a part of the body of a user who uses or puts on and takes off the dip-molded product. In the case where the object is a part of the body of a user, application of the powder to the surface of the dip-molded product can alleviate friction between the dip-molded product and the object. As a result, the feeling of use and attachability/detachability of the dip-molded product can be improved. However, the powder may cause allergy or infection. Thus, it is preferable to use no powder on the surface of the dip-molded product (without powder).

The molded article may be a multilayer dip-molded product having a multilayer structure in which a layer of the molded chloroprene copolymer rubber and a layer of a polymer other than the molded chloroprene copolymer rubber are layered. In this case, the layer of the molded chloroprene copolymer rubber is at least one layer of the multilayer structure. Examples of a polymer that can be used in the other layer(s) than the layer of the molded chloroprene copolymer rubber among the layers composing the multilayer structure include an isoprene homopolymer, a chloroprene homopolymer, an acrylonitrile-butadiene polymer, a butadiene polymer, polyvinyl chloride, and polyethylene. The molded article of the present invention may be used for any layer of the multilayer structure. When a polymer having smaller friction with the object than that of the molded chloroprene copolymer rubber is used for the layer to be in contact with the object, the friction between the multilayer dip-molded product and the object will be alleviated more, in comparison with a case where the layer of the molded chloroprene copolymer rubber comes in contact with the object. For example, in the case where the object is a part of the body of a user, the attachability/detachability of the multilayer dip-molded product is improved by use of a layer of a polymer having smaller friction with the body surface of the user than that of the molded chloroprene copolymer rubber as the layer to be contact with the body of the user in the multilayer dip-molded product. The multilayer dip-molded product may be produced by a known production method.

Examples

Hereinafter, the present invention will be further described in detail with reference to examples, but the present invention is not intended to be limited to these examples.

<Method for calculating polymerization conversion>

An emulsion was collected after the polymerization of the chloroprene copolymer (A) was started, and the collected emulsion was allowed to stand in an oven at 141°C for 30 minutes for drying to thereby provide a dried solid substance. The dried solid substance provided by the drying treatment includes a polymer and solid content other than the polymer. Then, the mass of the component that did not evaporate at 141°C among the various components used for the emulsion polymerization was calculated from the amount of the polymerization material fed, and was used as the mass of the solid content other than the polymer. A value obtained by subtracting the mass of the solid content other than the polymer from the mass of the dried solid substance provided by drying the emulsion after the polymerization was started was used as the "amount of the chloroprene copolymer (A) produced," and the polymerization conversion was calculated by the expression (1).

$$\text{Polymerization conversion (\% by mass)} = [(\text{amount of}$$

$$\text{chloroprene copolymer (A) produced})/(\text{mass of the total}$$

$$\text{monomers fed})] \times 100 \cdots (1)$$

The "mass of the total monomers fed" in the expression (1) is the total of the amount of 2-chloro-1,3-butadiene (A-1) fed, the amount of 2-methyl-1,3-butadiene (A-2) fed, and the amount of the optional monomers (A-3) fed included in the emulsion collected for providing the dried solid substance.

[Method for measuring physical properties of latex of chloroprene copolymer (A)]

As described below, after termination of the polymerization of the chloroprene copolymer (A), unreacted 2-chloro-1,3-butadiene (A-1), 2-methyl-1,3-butadiene (A-2), and optional monomers (A-3) were removed to provide a latex of the chloroprene copolymer (A). The various physical properties of the latex of the chloroprene copolymer (A) provided were evaluated by the following methods.

<Method for calculating solid content>

The latex of the chloroprene copolymer (A) was collected, and the mass of the collected latex of the chloroprene copolymer (A) was weighed. Thereafter, the weighed latex of the chloroprene copolymer (A) was allowed to stand in an oven at 141°C for 30 minutes for drying to thereby provide a dried solid substance. The solid content of the latex of the chloroprene copolymer (A) was calculated with the expression (2) from the mass of the latex of the chloroprene copolymer (A) before drying and the mass of the dried solid substance provided.

$$\text{Solid content (\% by mass)}$$

$$= [(\text{mass of dried solid substance})/(\text{mass of collected latex}$$

$$\text{of chloroprene copolymer (A)})] \times 100 \cdots (2)$$

<Tetrahydrofuran insoluble content of chloroprene copolymer (A) >

The tetrahydrofuran insoluble content of chloroprene copolymer (A) was measured as follows. Specifically, at 25°C, 1 g of a latex of the chloroprene copolymer (A) was added dropwise to 100 mL of tetrahydrofuran and shaken on a shaker (SA300) manufactured by Yamato Scientific Co., Ltd. for 10 hours. The mixture of the latex of the chloroprene copolymer (A) and tetrahydrofuran after the shaking treatment was subjected to separation by centrifugal sedimentation using a centrifugal sedimentation separator (manufactured by KOKUSAN Co. Ltd., H-9R) to provide a dissolution phase as a supernatant. The dissolution phase provided was heated to 100°C to evaporate the tetrahydrofuran over an hour, and the mass of the dried solid substance was measured. This provides the mass of the dissolved matters that were dissolved in the dissolution phase out of the chloroprene copolymer (A).

The mass of the chloroprene copolymer (A) in 1 g of the latex of the chloroprene copolymer (A) and the mass of the above dissolved matters were substituted into the expression (3) to calculate the tetrahydrofuran insoluble content that did not dissolve in tetrahydrofuran at 25°C out of the chloroprene copolymer (A).

```
Tetrahydrofuran insoluble content (% by mass)

= {1 - [(mass of dissolved matters)/(mass of chloroprene

copolymer (A) in 1 g of latex of chloroprene copolymer (A))]}

× 100···(3)
```

The mass of the chloroprene copolymer (A) in 1 g of the latex of the chloroprene copolymer (A) in the expression (3) was considered as the mass of the solid content provided by drying 1 g of the latex of the chloroprene copolymer (A) to solid. When the latex of the chloroprene copolymer (A) was dried to solid, the latex was allowed to stand in an oven at 141°C for 30 minutes for drying.

<Weight average molecular weight (Mw)>

An exemplary method for determining the weight average molecular weight (Mw) of the tetrahydrofuran soluble content at 25°C in the chloroprene copolymer (A) will be described below. In the same processing as for the sample preparation for the measurement of the tetrahydrofuran insoluble content described above, a dissolution phase as a supernatant after separation by centrifugal sedimentation was prepared, separated, and diluted with tetrahydrofuran to prepare a sample. The sample provided was subjected to molecular weight measurement in terms of polystyrene by GPC (gel permeation chromatography method) to measure the weight average molecular weight (Mw).

As for the GPC measurement conditions, LC-20AD manufactured by Shimadzu Corporation as a GPC measurement apparatus and RID-10A (refractive index detector) manufactured by Shimadzu Corporation as a detector were used. The type of column used was PLgel 10 $\mu$m MiniMIX-B manufactured by Agilent Technologies, Inc., the eluant was tetrahydrofuran (KANTO CHEMICAL CO., INC., for HPLC), the column temperature was 40°C, and the flow rate was 0.4 ml/min.

<Monomer unit content in chloroprene copolymer (A)>

The content of the component derived from 2-methyl-1,3-butadiene (A-2) in the chloroprene copolymer (A) was determined by $^1$H-NMR analysis. The latex of the chloroprene copolymer (A) was coagulated with methanol. After drying, deuterated chloroform was added to the coagulated product provided. The substance insoluble in deuterated chloroform was filtered off, and the solution provided was subjected to $^1$H-NMR analysis. For the $^1$H-NMR analysis, JNM-AL400 manufactured by JEOL Ltd was used as the measurement apparatus, and tetramethylsilane was used as a reference for the chemical shift.

The content of the component derived from 2-methyl-1,3-butadiene (A-2) was calculated from a peak (5.4 ppm) assigned to 2-chloro-1,3-butadiene (A-1) and a peak (5.1 ppm) assigned to 2-methyl-1,3-butadiene (A-2) in the $^1$H-NMR spectrum by the expression (4).

```
Content of component derived from 2-methyl-1,3-butadiene

(A-2) (mol%)

= (area of peak at 5.1 ppm)/(area of peak at 5.1 ppm + area

of peak at 5.4 ppm) × 100···(4)
```

When the monomer units derived from monomers (A-3) in the chloroprene copolymer (A) is contained but exhibits no peak overlapping the peak at 5.1 ppm or the peak at 5.4 ppm, the expression (4) can be used for determining the proportion of 2-methyl-1,3-butadiene (A-2) with respect to the total of 2-chloro-1,3-butadiene (A-1) and 2-methyl-1,3-butadiene (A-2).

When the proportion of the monomer (A-3) contained is determined, the proportion of the monomer (A-3) with respect to the total of the 2-chloro-1,3-butadiene (A-1) and the monomer (A-3) is calculated by an expression similar to the expression (4), by use of the peak area of peaks overlapping neither the peaks of 2-chloro-1,3-butadiene (A-1) nor 2-methyl-1,3-butadiene (A-2) among peaks assigned to the monomer (A-3). Similarly, the proportion of the monomers (A-3) is also determined with respect to 100 parts by mole of the total of the monomer units derived from 2-chloro-1,3-butadiene (A-1) and the monomer units derived from 2-methyl-1,3-butadiene (A-2).

When the monomer (A-3) exhibits peaks overlapping the peak at 5.1 ppm and the peak at 5.4 ppm, the respective peaks assigned to 2-chloro-1,3-butadiene (A-1), 2-methyl-1,3-butadiene (A-2), and the monomer (A-3) are identified using multidimensional NMR measurement results such as $^1$H-$^1$H COSY (COrrelation SpectroscopY), and the peak area can be used for the similar calculation to thereby determine the proportion of each substance.

[Example 1]

(1) Preparation of latex of chloroprene copolymer (A)

To a reaction vessel having an internal volume of 5 L, fed were 1,200 g of 2-chloro-1,3-butadiene (A-1), 300 g of 2-methyl-1,3-butadiene (A-2), 1,290 g of pure water, 65 g of disproportionated rosin acid (manufactured by Arakawa Chemical Industries, Ltd., R-600), 17.1 g of potassium hydroxide, 3.9 g of sodium hydroxide, 3.3 g of a sodium salt of a β-naphthalenesulfonic acid-formalin condensate, and 1.65 g of n-dodecylmercaptan. The starting materials fed in the reaction vessel were emulsified, and the rosin acid was converted into rosin acid soap.

2-chloro-1,3-butadiene (A-1) and 2-methyl-1,3-butadiene (A-2) were blended as starting material monomers, and pure water was blended as a dispersion medium for emulsion polymerization. The disproportionated rosin acid, potassium hydroxide, and sodium hydroxide were blended as materials for an emulsifier, and the sodium salt of a β-naphthalenesulfonic acid-formalin condensate was blended as an emulsifier.

To an emulsion provided by emulsifying the starting materials, 4 g of potassium persulfate was added as a polymerization initiator, and emulsion polymerization was conducted under a nitrogen gas atmosphere at 30°C. When the polymerization conversion of all the monomers reached 84% by mass (after 6.1 hours), the polymerization was terminated. The method for calculating the polymerization conversion is as described above.

Subsequently, unreacted 2-chloro-1,3-butadiene (A-1) and 2-methyl-1,3-butadiene (A-2) were removed by steam distillation to provide a latex of the chloroprene copolymer (A1). The physical properties of the latex of the chloroprene copolymer (A1) are as follows.

Solid content: 45% by mass
Tetrahydrofuran insoluble content: 2% by mass
Weight average molecular weight (Mw) of the tetrahydrofuran soluble content at 25°C of the chloroprene copolymer (A1): 600,000
Proportion of the monomer units derived from 2-methyl-1,3-butadiene (A-2) in the chloroprene copolymer (A1): 15 mol%

(2) Preparation of chloroprene copolymer latex composition

The latex of the chloroprene copolymer (A1) provided in (1) described above was fed along with the vulcanization accelerator (B), the metal oxide (C), the sulfur (D), and the antioxidant (E) into a vessel equipped with a stirrer. The amount of the vulcanization accelerator (B) fed was as follows: 0.5 parts by mass of zinc 2-mercaptobenzothiazole (NOCCELER(registered trademark) MZ manufactured by Ouchi Shinko Chemical Industrial Co., Ltd.), 0.5 parts by mass of zinc dibutyldithiocarbamate (NOCCELER(registered trademark) BZ manufactured by Ouchi Shinko Chemical Industrial Co., Ltd.), and 1.0 part by mass of sodium dibutyldithiocarbamate (NOCCELER(registered trademark) TP manufactured by Ouchi Shinko Chemical Industrial Co., Ltd.), per 100 parts by mass of the solid content in the latex of the chloroprene copolymer (A1). The amount of each of the metal oxide (C), the sulfur (D), and the antioxidant (E) fed was as follows: 0.5 parts by mass of zinc oxide (AZ-SW manufactured by Osaki Industry Co., Ltd.), 1.5 parts by mass of sulfur (S-50 manufactured by Nippon Color Ind. Co., Ltd.), and 2.0 parts by mass of a phenol-based antioxidant (K-840 manufactured by Chukyo Yushi Co., Ltd.), per 100 parts by mass of the solid content in the latex of the chloroprene copolymer (A1). The mixture fed in the vessel equipped with a stirrer was homogeneously mixed by stirring for 20 minutes to thereby provide a chloroprene copolymer latex composition. The chloroprene copolymer latex composition after stirring was allowed to stand at 23°C for 20 hours for maturing.

The zinc oxide AZ-SW, sulfur S-50, and phenolic antioxidant K-840 were each in the form of a dispersion, which includes the zinc oxide, the sulfur (D), or the antioxidant (E) as an active ingredient dispersed in a liquid medium. The amount of each of the above-described zinc oxide AZ-SW, sulfur S-50, and phenolic antioxidant K-840 fed is only the amount of the active ingredient of each of the zinc oxide AZ-SW, the sulfur S-50, and the K-840 fed.

(3) Production of film

The chloroprene copolymer latex composition provided in the above (2) was used to obtain a molded article (film) of a chloroprene copolymer rubber by the dip processing method.

As a mold for the film of the chloroprene copolymer, a ceramic plate of 200 mm in length, 100 mm in width, and 5 mm in thickness was provided. This mold was dipped in a 30% by mass calcium nitrate aqueous solution, then withdrawn, and dried in an oven at 40°C for 10 minutes to thereby cause calcium nitrate, as a coagulant, to adhere to the surface of the mold.

Further, the dried mold was dipped in the chloroprene copolymer latex composition provided in the above (2) to cause the solid content of the chloroprene copolymer latex composition to deposit on the surface of the mold. The mold was withdrawn from the chloroprene copolymer latex composition and then dried in an oven at 70°C for 30 minutes.

Next, the mold with the solid content deposited on the surface thereof was heated in an oven at 110°C for 90 minutes to cure the solid content of the chloroprene copolymer latex composition deposited on the surface of the mold by vulcanization treatment. After left to cool under atmospheric air, the molded article cured on the surface of the mold was cut into a desired shape and size to thereby provide a film as a molded article of the vulcanized chloroprene copolymer rubber.

[Thermal degradation treatment of molded article, and method for measuring physical properties of molded article]

The film was cut with the No. 6 dumbbell specified in JIS K6251-2017 to provide a specimen. The specimen has a thickness of 0.15 to 0.25 mm.

<Tensile strength, tensile elongation ratio, and elastic modulus>

The specimen was subjected to a tensile test at 23°C by a method in accordance with JIS K6251-2017, and thus, the tensile strength, the tensile elongation ratio, the elastic modulus at 100% elongation (100% elastic modulus), and the elastic modulus at 500% elongation (500% elastic modulus) were measured. The various physical properties of the film measured as described above are summarized in Table 1.

Example 2:

A chloroprene copolymer latex composition, a film, and a specimen were produced in the same manner as in Example 1 except that zinc dibutyldithiocarbamate (NOCCELER(registered trademark) BZ manufactured by Ouchi Shinko Chemical Industrial Co., Ltd.) in the vulcanization accelerator (B) was replaced by zinc diethyldithiocarbamate (NOCCELER(registered trademark) EZ manufactured by Ouchi Shinko Chemical Industrial Co., Ltd.). Various evaluations were conducted in the same manner as in Example 1. The results are shown in Table 1.

[Example 3]

A chloroprene copolymer latex composition, a film, and a specimen were produced in the same manner as in Example 1 except that the amount of zinc oxide blended was changed to 5.0 parts by mass and further that the vulcanization time was changed to 20 minutes. Various evaluations were conducted in the same manner as in Example 1. The results are shown in Table 1.

[Comparative Example 1]

A chloroprene copolymer latex composition, a film, and a specimen were produced in the same manner as in Example 1 except that 1.0 part by mass of zinc 2-mercaptobenzothiazole (NOCCELER(registered trademark) MZ manufactured by Ouchi Shinko Chemical Industrial Co., Ltd.) was used singly as the vulcanization accelerator (B) and that the vulcanization time was set to 20 minutes. Various evaluations were conducted in the same manner as in Example 1. The results are shown in Table 1.

[Comparative Example 2]

A chloroprene copolymer latex composition, a film, and a specimen were produced in the same manner as in Example 1 except that 1.0 part by mass of zinc dibutyldithiocarbamate (NOCCELER(registered trademark) BZ manufactured by Ouchi Shinko Chemical Industrial Co., Ltd.) was used singly as the vulcanization accelerator (B) and that the vulcanization time was set to 20 minutes. Various evaluations were conducted in the same manner as in Example 1. The results are shown in Table 1.

[Comparative Example 3]

A chloroprene copolymer latex composition, a film, and a specimen were produced in the same manner as in Example 1 except that 1.0 part by mass of diphenyl guanidine (NOCCELER(registered trademark) D manufactured by Ouchi Shinko Chemical Industrial Co., Ltd.) was used singly as the vulcanization accelerator (B) and that the vulcanization time was set to 20 minutes. Various evaluations were conducted in the same manner as in Example 1. The results are shown in Table 1.

[Comparative Example 4]

A chloroprene copolymer latex composition, a film, and a specimen were produced in the same manner as in Example 1 except that 1.0 part by mass of diphenyl guanidine (NOCCELER(registered trademark) D manufactured by Ouchi Shinko Chemical Industrial Co., Ltd.) and 1.0 part of N,N'-diphenylthiourea (NOCCELER(registered trademark) C manufactured by Ouchi Shinko Chemical Industrial Co., Ltd.) were used as the vulcanization accelerator (B) and that the vulcanization time was changed to 20 minutes. Various evaluations were conducted in the same manner as in Example 1. The results are shown in Table 1.

[Comparative Example 5]

A chloroprene copolymer latex composition, a film, and a specimen were produced in the same manner as in Example 1 except that 1,500 g of 2-chloro-1,3-butadiene (A-1) was used as the monomer fed in the reaction vessel on preparation of the latex of the chloroprene copolymer and that no 2-methyl-1,3-butadiene (A-2) was used. Various evaluations were conducted in the same manner as in Example 1. The results are shown in Table 1.

[Comparative Example 6]

A chloroprene copolymer latex composition, a film, and a specimen were produced in the same manner as in Example 1 except that, in the vulcanization accelerator (B) used in the Example 1, 1.0 part by mass of sodium dibutyldithiocarbamate ((NOCCELER(registered trademark) TP manufactured by Ouchi Shinko Chemical Industrial Co., Ltd.) was replaced by 0.25 parts by mass of diphenylguanidine (NOCCELER(registered trademark) D manufactured by Ouchi Shinko Chemical Industrial Co., Ltd.) and that the vulcanization time was changed to 20 minutes. Various evaluations were conducted in the same manner as in Example 1. The results are shown in Table 1.

[Table 1]

| | | Example | | | Comparative Example | | | | | |
|---|---|---|---|---|---|---|---|---|---|---|
| | | 1 | 2 | 3 | 1 | 2 | 3 | 4 | 5 | 6 |
| Polymerization conditions | Amount of 2-chloro-1,3-butadiene (A-1) used (parts by mass) [mol%] | 80 [75.5] | 80 [75.5] | 80 [75.5] | 80 [75.5] | 80 [75.5] | 80 [75.5] | 80 [75.5] | 100 [100] | 80 [75.5] |
| | Amount of 2-methyl-1,3-butadiene (A-2) used (parts by mass) [mol%] | 20 [24.5] | 20 [24.5] | 20 [24.5] | 20 [24.5] | 20 [24.5] | 20 [24.5] | 20 [24.5] | 0 [0] | 20 [24.5] |
| | Polymerization conversion (% by mass) | 84 | 84 | 84 | 84 | 84 | 84 | 84 | 90 | 84 |
| Physical properties of latex | Proportion of monomer units derived from 2-methyl-1,3-butadiene in copolymer (mol%) | 15 | 15 | 15 | 15 | 15 | 15 | 15 | 0 | 15 |
| | Solid content (% by mass) | 45 | 45 | 45 | 45 | 45 | 45 | 45 | 48 | 45 |
| | Tetrahydrofuran insoluble content (% by mass) | 2 | 2 | 2 | 2 | 2 | 2 | 2 | 0 | 2 |
| | Weight average molecular weight (Mw) ($\times 10^5$) | 6.0 | 6.0 | 6.0 | 6.0 | 6.0 | 6.0 | 6.0 | 7.3 | 6.0 |
| Formulation | Zinc 2-mercaptobenzothiazole (parts by mass) | 0.5 | 0.5 | 0.5 | 1.0 | 0 | 0 | 0 | 0.5 | 0.5 |
| | Zinc dibutyldithiocarbamate (parts by mass) | 0.5 | 0 | 0.5 | 0 | 1.0 | 0 | 0 | 0.5 | 0.5 |
| | Zinc diethyldithiocarbamate (parts by mass) | 0 | 0.5 | 0 | 0 | 0 | 0 | 0 | 0 | 0 |
| | Sodium dibutyldithiocarbamate (parts by mass) | 1.0 | 1.0 | 1.0 | 0 | 0 | 0 | 0 | 1.0 | 0 |
| | Diphenylguanidine (parts by mass) | 0 | 0 | 0 | 0 | 0 | 1.0 | 1.0 | 0 | 0.25 |
| | N,N'-diphenylthiourea (parts by mass) | 0 | 0 | 0 | 0 | 0 | 0 | 1.0 | 0 | 0 |
| | Zinc oxide (parts by mass) | 0.5 | 0.5 | 5.0 | 0.5 | 0.5 | 0.5 | 0.5 | 0.5 | 0.5 |
| | Sulfur (parts by mass) | 1.5 | 1.5 | 1.5 | 1.5 | 1.5 | 1.5 | 1.5 | 1.5 | 1.5 |
| Conditions for vulcanization | Vulcanization temperature (°C) | 110 | 110 | 110 | 110 | 110 | 110 | 110 | 110 | 110 |
| | Vulcanization time (minutes) | 90 | 90 | 20 | 20 | 20 | 20 | 20 | 90 | 20 |
| Physical properties after vulcanization | 100% elastic modulus (MPa) | 0.59 | 0.58 | 0.6 | - | - | - | 0.52 | - | 0.59 |
| | 500% elastic modulus (MPa) | 1.49 | 1.52 | 1.52 | - | - | - | 1.17 | - | 1.32 |
| | Tensile strength (MPa) | 19.7 | 19.1 | 19.5 | - | - | - | 15.5 | - | 19.3 |
| | Tensile elongation ratio (%) | 950 | 1000 | 1050 | - | - | - | 1150 | - | 1050 |

In Examples 1 to 3, in which both the thiazole-based vulcanization accelerator and the carbamate-based vulcanization accelerator were used as the vulcanization accelerator (B) in the chloroprene copolymer latex composition, the films after vulcanization treatment exhibited high flexibility and strength. The flexibility and strength of the films provided in Examples 1 to 3 are comparable to the results in Comparative Example 6, in which diphenylguanidine capable of achieving favorable vulcanization treatment was used. In other words, use of both the thiazole-based vulcanization accelerator and the carbamate-based vulcanization accelerator as the vulcanization accelerator (B) enabled production of a molded article having desired flexibility and strength without use of a vulcanization accelerator that is feared to have skin sensitization (diphenylguanidine). It is considered that this result is given because of the following: when using the thiazole-based vulcanization accelerator, crosslinked structures is formed as soon as the vulcanization temperature is reached; and when using the carbamate-based vulcanization accelerator, formation of crosslinked structures is started after the passage of a certain period from reaching the vulcanization temperature. In other words, it is considered that crosslinked structures are formed by the thiazole-based vulcanization accelerator in the early stage of the vulcanization treatment, and that formation of crosslinked structures occurs due to the carbamate-based vulcanization accelerator even when the thiazole-based vulcanization accelerator is deactivated so that crosslinked structures are continuously formed during the vulcanization treatment period.

On the other hand, in Comparative Examples 1 and 2, in which either one of the thiazole-based vulcanization accelerator or the carbamate-based vulcanization accelerator was used alone, removal of the film after the vulcanization treatment was failed, and thus the physical properties of the film after the vulcanization treatment could not be evaluated. It is considered that this result is given because of the following: in Comparative Example 1, in which only the thiazole-based vulcanization accelerator was used, crosslinked structures were formed only in the early stage of the vulcanization treatment; and in Comparative Example 2, in which only the carbamate-based vulcanization accelerator was used, crosslinked structures were formed only after the carbamate-based vulcanization accelerator was activated.

Also in Comparative Example 3, in which a guanidine-based vulcanization accelerator was used alone as the vulcanization accelerator, the physical properties of the film after the vulcanization treatment could not be evaluated. The molded article provided in Comparative Example 4, in which a guanidine-based vulcanization accelerator and thiourea-based vulcanization accelerator were used in combination, has insufficient tensile strength for surgical gloves. Also in Comparative Example 5, in which only 2-chloro-1,3-butadiene (A-1) was included as the monomer units of the chloroprene copolymer, the physical properties of the film after the vulcanization treatment could not be evaluated.

## Claims

1. A chloroprene copolymer latex composition comprising a chloroprene copolymer (A) and a vulcanization accelerator (B), wherein

   the chloroprene copolymer (A) is a chloroprene copolymer comprising monomer units derived from chloroprene and from 2-methyl-1,3-butadiene, and
   the vulcanization accelerator (B) comprises a carbamate-based vulcanization accelerator and a thiazole-based vulcanization accelerator.

2. The chloroprene copolymer latex composition according to claim 1, wherein the thiazole-based vulcanization accelerator is zinc 2-mercaptobenzothiazole.

3. The chloroprene copolymer latex composition according to claim 1 or 2, wherein the carbamate-based vulcanization accelerator is at least one selected from zinc diethyldithiocarbamate, zinc dibutyldithiocarbamate, and sodium dibutyldithiocarbamate.

4. The chloroprene copolymer latex composition according to any one of claims 1 to 3, wherein the chloroprene copolymer latex composition further comprises a metal oxide (C), sulfur (D), and an antioxidant (E).

5. A chloroprene copolymer latex composition according to claim 4, comprising:

   100 parts by mass of the chloroprene copolymer (A) and an optional synthetic rubber (F) in total;
   0.1 to 10.0 parts by mass of the vulcanization accelerator (B);
   0.1 to 20.0 parts by mass of the metal oxide (C);
   0.1 to 10.0 parts by mass of the sulfur (D); and
   0.1 to 10.0 parts by mass of the antioxidant (E).

6. The chloroprene copolymer latex composition according to claim 5, comprising a synthetic rubber (F), wherein a proportion of the synthetic rubber (F) is 1 to 33% by mass with respect to 100% by mass of a total of the chloroprene copolymer (A) and the synthetic rubber (F).

7. A molded article of chloroprene copolymer rubber, provided by curing the chloroprene copolymer latex composition according to any one of claims 1 to 6.

8. A dip-molded product provided by molding the chloroprene copolymer latex composition according to any one of claims 1 to 6 by a dipping method followed by curing.

9. The dip-molded product according to claim 8, wherein the dip-molded product is gloves.

10. The dip-molded product according to claim 9, wherein the dip-molded product is medical disposable gloves.

11. The dip-molded product according to any one of claims 8 to 10, wherein no powder is present on a surface of the dip-molded product, the powder being for alleviating friction between the dip-molded product and an object to be in contact with the dip-molded product.

12. A multilayer dip-molded product having a multilayer structure comprising:

    a layer provided by molding the chloroprene copolymer latex composition according to any one of claims 1 to 6 by a dipping method followed by curing; and
    another layer thereon.

## INTERNATIONAL SEARCH REPORT

| International application No. |
|---|
| PCT/JP2020/048437 |

### A. CLASSIFICATION OF SUBJECT MATTER

C08L 11/02(2006.01)i; A41D 19/00(2006.01)i; A41D 19/04(2006.01)i; A61B 42/10(2016.01)i; B29C 41/14(2006.01)i; B29C 41/36(2006.01)i; C08F 236/18(2006.01)i; C08K 3/06(2006.01)i; C08K 3/22(2006.01)i; C08K 5/39(2006.01)i; C08K 5/47(2006.01)i

FI:     C08L11/02; C08K5/39; C08K5/47; C08K3/06; C08K3/22; C08F236/18; B29C41/14; B29C41/36; A41D19/00 P; A41D19/04 B; A61B42/10

According to International Patent Classification (IPC) or to both national classification and IPC

### B. FIELDS SEARCHED

Minimum documentation searched (classification system followed by classification symbols)

C08L1/00-101/14; C08K3/00-13/08; C08F6/00-246/00;301/00; A41D19/00; A41D19/04; A61B42/10; B29C41/14; B29C41/36

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2003-055409 A (DENKI KAGAKU KOGYO KABUSHIKI KAISHA) 26 February 2003 (2003-02-26) entire text | 1-12 |
| A | JP 2009-185136 A (DENKI KAGAKU KOGYO KABUSHIKI KAISHA) 20 August 2009 (2009-08-20) entire text | 1-12 |
| A | JP 2019-147969 A (ANSELL LIMITED) 05 September 2019 (2019-09-05) entire text | 1-12 |
| A | WO 2017/154736 A1 (ZEON CORP.) 14 September 2017 (2017-09-14) entire text | 1-12 |
| A | JP 2008-056858 A (YAMASEI CORPORATION) 13 March 2008 (2008-03-13) entire text | 1-12 |
| A | WO 2013/099501 A1 (ZEON CORP.) 04 July 2013 (2013-07-04) entire text | 1-12 |

☐ Further documents are listed in the continuation of Box C.      ☒ See patent family annex.

| | |
|---|---|
| *    Special categories of cited documents: | "T"    later document published after the international filing date or priority date and not in conflict with the application but cited to understand the principle or theory underlying the invention |
| "A"    document defining the general state of the art which is not considered to be of particular relevance | |
| "E"    earlier application or patent but published on or after the international filing date | "X"    document of particular relevance; the claimed invention cannot be considered novel or cannot be considered to involve an inventive step when the document is taken alone |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
|---|---|
| 04 March 2021 (04.03.2021) | 16 March 2021 (16.03.2021) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

INTERNATIONAL SEARCH REPORT
Information on patent family members

International application no.

PCT/JP2020/048437

| Patent Documents referred in the Report | Publication Date | Patent Family | Publication Date |
|---|---|---|---|
| JP 2003-055409 A | 26 Feb. 2003 | (Family: none) | |
| JP 2009-185136 A | 20 Aug. 2009 | (Family: none) | |
| JP 2019-147969 A | 05 Sep. 2019 | JP 2017-508840 A<br>US 2015/0272241 A1<br>entire text<br>US 2019/0218375 A1<br>WO 2015/143473 A1<br>EP 3122816 A1<br>CN 106459515 A | |
| WO 2017/154736 A1 | 14 Sep. 2017 | US 2019/0031861 A1<br>entire text<br>EP 3428217 A1<br>CN 108779257 A<br>KR 10-2018-0124852 A | |
| JP 2008-056858 A | 13 Mar. 2008 | (Family: none) | |
| WO 2013/099501 A1 | 04 Jul. 2013 | JP 2012-62487 A<br>US 2015/0087761 A1<br>entire text<br>EP 2799483 A1<br>TW 201336935 A | |

Form PCT/ISA/210 (patent family annex) (January 2015)

**REFERENCES CITED IN THE DESCRIPTION**

**Patent documents cited in the description**

- JP 2019143002 A

- JP 2019044116 A